# EUROPEAN PATENT SPECIFICATION

(11) **EP 4 513 165 B1**
(45) Date of publication and mention of the grant of the patent: **26.08.2026**
(21) Application number: 23811876.4
(22) Date of filing: 25.05.2023
(51) Int. Cl.: G01N 3/20, G01N 3/08, G01N 17/00

(54) **METHOD FOR ASSESSING DELAYED FRACTURE CHARACTERISTICS OF SHEARED END SURFACE AND METHOD FOR PRODUCING AUTOMOTIVE COMPONENTS**
VERFAHREN ZUR BEURTEILUNG VERZÖGERTER BRUCHEIGENSCHAFTEN EINER GESCHERTEN ENDFLÄCHE UND VERFAHREN ZUR HERSTELLUNG VON AUTOMOBILKOMPONENTEN
PROCÉDÉ D'ÉVALUATION DE CARACTÉRISTIQUES DE RUPTURE DIFFÉRÉE DE SURFACE D'EXTRÉMITÉ CISAILLÉE ET PROCÉDÉ DE PRODUCTION DE COMPOSANTS AUTOMOBILES

(30) Priority: 25.05.2022 JP 2022085550
(43) Date of publication of application: 26.02.2025
(73) Proprietor: JFE Steel Corporation, Tokyo 100-0011 (JP)
(72) Inventor: MATSUKI, Yuichi, Tokyo 100-0011 (JP); SHINMIYA, Toyohisa, Tokyo 100-0011 (JP); NAKAGAWA, Kinya, Tokyo 100-0011 (JP); YAMASAKI, Yuji, Tokyo 100-0011 (JP)
(74) Representative: Haseltine Lake Kempner LLP
(86) International application number: PCT/JP2023/019488
(87) International publication number: WO 2023/229004

(56) References cited:
- EP-A1- 3 939 712
- EP-A1- 4 239 313
- JP-A- 2010 107 297
- JP-A- 2022 014 784
- JP-A- 2022 024 814
- JP-B1- 7 004 126

## Description

### Technical Field

The present invention relates to a technology related to an assessment of delayed fracture characteristics of a sheared end surface of a metal sheet, and a method for producing automotive components including the technology related to the assessment.

Herein, an end surface obtained by applying shearing processing to a metal sheet is referred to as the sheared end surface. The present invention is a technology suitable particularly for high-strength steel sheets having a tensile strength of 980 MPa or more (high-tensile strength steel sheets). In this specification, steel sheets having a tensile strength of 1470 MPa or more among the high-strength steel sheets are referred to as ultrahigh-strength steel sheets.

### Background Art

At present, automobiles have been required to improve fuel consumption by a reduction in weight and collision safety. For the purpose of achieving both the reduction in weight of a vehicle body and the protection of passengers in the event of a collision, the high-strength steel sheets are used for the vehicle body. Particularly in recent years, the high-strength steel sheets having a tensile strength of 980 MPa or more have begun to be applied to the vehicle body. As one of the problems when the high-strength steel sheets are applied to the vehicle body, a delayed fracture is mentioned. Particularly in the high-strength steel sheets having a tensile strength of 980 MPa or more, the delayed fracture occurring from the sheared end surface is a significant problem. The sheared end surface is an end surface after shearing processing. The above-described problem is particularly problematic in the ultrahigh-strength steel sheets having a tensile strength 1470 MPa or more among the high-strength steel sheets.

Herein, it is known that a large tensile stress remains in the sheared end surface. The remaining of the large tensile stress raises a concern that the delayed fracture occurs in the metal sheet.

To predict the delayed fracture in the sheared end surface in advance, it is necessary to prepare a test piece for assessment and place the test piece in a hydrogen entry environment. Further, the sheared end surface has properties changing by plastic deformation in shearing processing. In general, a risk of the delayed fracture in the sheared end surface increases. Thus, PTL 1, for example, assesses the occurrence of the delayed fracture as follows. More specifically, PTL 1 applies compression processing in the sheet thickness direction by rolling to the sheared end surface of the test piece. Thereafter, the test piece is placed in a hydrogen entry environment, and the occurrence of the delayed fracture is assessed.

Herein, a test is supposed in which the sheared end surface kept as-sheared is placed in a hydrogen entry environment under no load. Even when the delayed fracture does not occur in this test, the delayed fracture sometimes occurs when the test is performed while a stress is being applied from the outside. This is because a load stress from the outside is added to the large tensile stress remaining in the sheared end surface. Therefore, in PTL 2, for example, a constant load by tension is loaded to an assessment sample including the sheared end surface, the assessment sample is placed in a hydrogen entry environment in a restrained state, and the delayed fracture characteristics are assessed. As a more simplified method, in PTL 3, a test piece is placed in a hydrogen environment in a state where a load by bending is being loaded to the test piece, and the delayed fracture characteristics are assessed. However, in PTL 3, the sheared end surface is not targeted, and the principal object is to assess the delayed fracture characteristics in the front surface of the test piece. Therefore, in PTL 3, the front surface of the sheared end surface of an assessment sample is sealed by a resin coating, and the sheared end surface is excluded from an assessment target.

The present inventors have conducted various examinations and obtained the following findings. More specifically, the present inventors have obtained a finding that there is another problem with prediction or prevention of the occurrence of the delayed fracture based on these delayed fracture assessment techniques for actual automotive components.

For example, the introduction of a strain by rolling as in PTL 1 has the following problem. More specifically, there is such a problem that the introduction of a strain by rolling as in PTL 1 deviates from a deformation state in a forming strain introduced by press forming, which is used for automotive components. In the press forming, uniaxial tension and compression and a bending deformation by a combination of the uniaxial tension and compression are introduced into the sheared end surface. Therefore, the assessment technique as in PTL 1 does not achieve sufficient assessment. PTLS 2, 3 do not consider changes in the delayed fracture characteristics by plastic deformation after shearing processing of the sheared end surface. Therefore, the assessment is not sufficient as a delayed fracture assessment in press formed articles where various forming strains are generated in the sheared end surface.

In all the assessment methods of PTLS 1 to 3, the occurrence or non-occurrence and time of the delayed fracture under laboratory-like individual hydrogen entry conditions and stress conditions were merely assessed.

Reference may also be made to JP 7004126 B1, which relates to a delayed fracture characteristic evaluation method and program.

### Citation List

### Patent Literatures

PTL 1: JP 2020-41837 A
PTL 2: JP 5196926 B
PTL 3: JP 5971058 B

### Summary of Invention

### Technical Problem

Conventionally, an assessment in the following viewpoint has not been conducted. The viewpoint is the degree of margin in the conditions of the hydrogen entry environment or the stress with respect to the occurrence of the delayed fracture, as compared with the hydrogen entry environment or the stress in actual automotive components.

Then, the present inventors have obtained the following finding. More specifically, in the actual automotive components, forming strains different among the formation places of the sheared end surfaces are introduced into a metal sheet to be processed. The present inventors have obtained a finding that the forming strain causes a change in the delayed fracture characteristics by plastic deformation. Further, in the sheared end surface, a forming residual stress after press forming is added to a residual stress by shearing, so that the delayed fracture is likely to occur.

Further, the present inventors have obtained the following finding. More specifically, a case is supposed in which a forming residual stress is loaded to the sheared end surface into which a forming strain is introduced in a certain hydrogen entry environment. In this case, the present inventors have obtained a finding that it is very important to assess the degree of margin to the occurrence of the delayed fracture in the sheared end surface of a press formed article. More specifically, the present inventors have obtained a finding that such an assessment is very important in avoiding the delayed fracture in the sheared end surfaces in automotive components.

As described above, the sheared end surface properties change by plastic deformation by press forming in automotive components. Conventionally, there has been no index that enables the prediction of the occurrence of the delayed fracture as compared with stresses that are generated in actual automotive components. Therefore, there has been no technique by which the delayed fracture can be assessed from the viewpoint of a stress margin.

The present invention focuses on the above-described point and aims to enable a more accurate assessment of the delayed fracture characteristics in the sheared end surface.

### Solution to Problem

The present invention is defined by the appended claims. One aspect of the present invention provides a method for assessing delayed fracture characteristics for assessing the delayed fracture characteristics of a sheared end surface of a metal sheet, the method including: a test including a step of restraining a metal sheet in a state where a predetermined load stress is loaded to a sheared surface of the metal sheet and a step of placing the metal sheet for a predetermined time in a predetermined hydrogen entry environment in the restrained state; and a step of determining a limit load stress being a load stress at the limit where a delayed fracture in the sheared surface of the metal sheet does not occur based on the results of the test and setting a stress margin to the occurrence of the delayed fracture in the sheared end surface of the metal sheet based on the determined limit load stress, in which the determined stress margin is set as an index of the assessment of the delayed fracture characteristics of the sheared end surface of the metal sheet. The test includes, before the restraining step, a step of applying a forming strain along the extension direction of the sheared surface to the sheared surface of the metal sheet. The stress margin is set as a value with the forming strain as a variable. In the step of applying the forming strain, the forming strain is applied by uniaxial tension or uniaxial compression

### Advantageous Effects of Invention

The aspect of the present invention enables the application of an index for more accurately assessing the delayed fracture occurring in the sheared end surface.

At this time, the index (stress margin) has a stress as a unit and enables the assessment from the viewpoint of the margin by stress. Therefore, when the high-strength steel sheets are applied to various components, such as panel components and structural and frame components, of automobiles, for example, the following can be achieved. More specifically, the aspect of the present invention enables the prediction of the occurrence of the delayed fracture, including the margin having a stress dimension.

The aspect of the present invention enables a reduction in weight of an automobile body by expanding the application range of ultrahigh-strength steel sheets, for example.

### Brief Description of Drawings

FIG. 1 is a view illustrating an example of an assessment procedure of a stress margin corresponding to the amount of strain using a test piece;
FIG. 2 is a view illustrating an example of a stress margin with a forming strain as a variable;
FIGS. 3A and 3B are conceptual views each illustrating the relation between a delayed fracture and the stress margin of a sheared end surface;
FIGS. 4A and 4B are conceptual views each illustrating the relation between a delayed fracture and the stress margin of a sheared end surface when a residual stress remains in bending forming;
FIG. 5 is an explanatory view illustrating an example of a method for loading a load stress by bending;
FIG. 6 is a view illustrating an example of a function for the amount of strain of the stress margin;
FIG. 7 is a view illustrating an example of a delayed fracture determination using the stress margin; and
FIG. 8 is a view illustrating an example of the flow of a program that can be used in the assessment in this technique.

### Description of Embodiments

Next, embodiments of the present invention are described with reference to the drawings.

### (Configuration)

This embodiment is a method for assessing delayed fracture characteristics for assessing the delayed fracture characteristics of a sheared end surface of a metal sheet. The present invention further exhibits the effects particularly when the metal sheet is a high-strength steel sheet.

As an assessment index for the method for assessing delayed fracture characteristics, a "stress margin", which is an index newly set in the present disclosure, is determined.

The "stress margin" in the present disclosure is the allowance of an external load stress, in which a delayed fracture does not occur, of the sheared end surface.

This embodiment uses the method for assessing delayed fracture characteristics of this embodiment when automotive components are produced by applying processing, such as bending processing, to a metal sheet having a sheared cross section, for example. For example, the delayed fracture characteristics in the sheared end surface are assessed by the method for assessing delayed fracture characteristics of this embodiment, and the occurrence of the delayed fracture in the automotive components to be produced is predicted. Then, the shapes, materials, and the like of the automotive components with less occurrence of the delayed fracture, for example, are selected based on the prediction, and the automotive components are produced under the selected conditions.

In the following embodiment, the stress margin is set as a value in which a forming strain, which is one parameter of test conditions, is set as a variable. The forming strain is a strain applied before the external load stress is loaded. The stress margin is not limited to a case where the forming strain is set as a variable. The stress margin is preferably set such that parameters of the production conditions of the metal sheet before the external load stress is loaded to the metal sheet are set as variables because the application range increases. The parameters as used herein include a forming strain, a clearance in shearing, wear conditions (shearing processing conditions), and the like, for example. The shearing processing condition is one of the processing conditions of a material to be assessed.

The stress margin may be expressed as a value in which test conditions other than a step (third step 12) of loading an external load to a metal sheet and restraining the metal sheet are set as variables (parameters).

Herein, the test conditions other than the step of loading an external load to a metal sheet and restraining the metal sheet include the following test conditions, for example. More specifically, examples of the test conditions include material types (steel grade and sheet thickness), shearing conditions (clearance and wear conditions), conditions in a hydrogen environment for immersion (time of immersion), and the like. The reason for excluding test conditions of the restraining step is to determine a limit load stress by changing the parameters thereof.

The stress margin may be a value obtained by multiplying the limit load stress by a predetermined safety factor.

Herein, the external load stress described above is a load stress generated when a metal sheet is press-formed into a desired product shape or when the product is assembled.

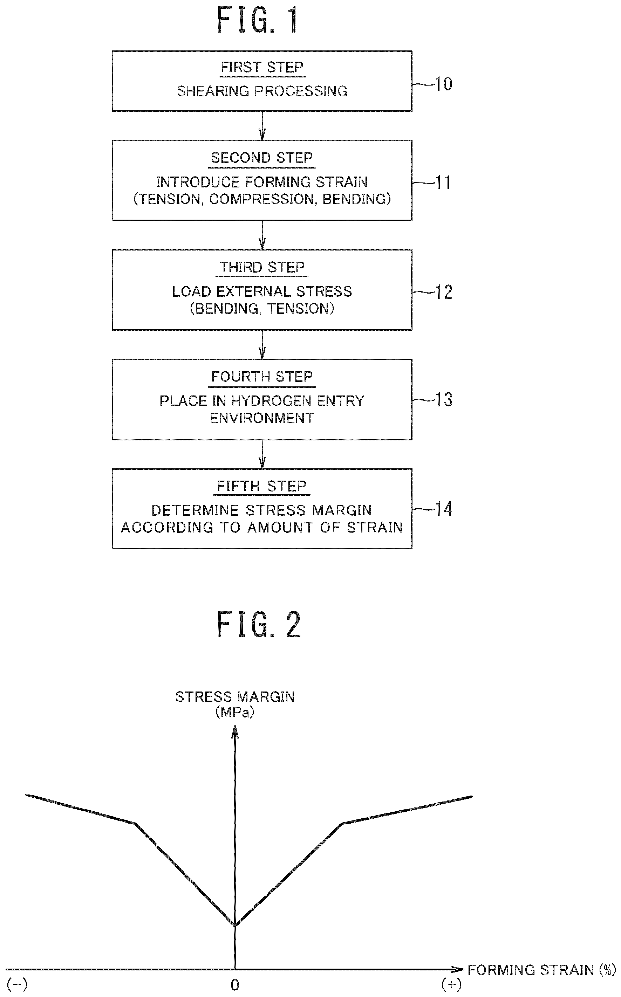

This embodiment includes the following step as processing for determining the stress margin of a metal sheet to be assessed. More specifically, this embodiment includes a test step having an actual experiment and a step of setting the stress margin. Specifically, this embodiment includes steps of a first step 10 to a fifth step 14 as illustrated in FIG. 1.

In FIG. 1, the first step 10 to the fourth step 13 correspond to the test step. The fifth step 14 corresponds to the step of setting the stress margin.

### <First step 10>

The first step 10 is a step of preparing a test piece from a metal sheet having the same conditions as those of the metal sheet to be assessed. In the first step 10, shearing processing is applied to a metal sheet containing the same material and having the same thickness as those of the metal sheet to be assessed. Then, the test piece having a sheared end surface for determining the stress margin is prepared.

### <Second step 11>

The second step 11 is a step of applying a forming strain to at least a part of the sheared end surface of the test piece. The forming strain to be applied is a strain along the extension direction of the sheared end surface.

The forming strain to be applied is 0.1% or more, for example.

The application of the forming strain is carried out by applying uniaxial tension or uniaxial compression to the test piece, for example. The application of the forming strain is carried out by bending the test piece in the sheet thickness direction, for example.

### <Third step 12>

The third step 12 is a step of loading a predetermined external load stress to the sheared end surface of the test piece and restraining the test piece in the loaded state. A method for loading a stress is performed by loading a tensile stress or loading a bending stress, for example. In this case, a method for loading a bending stress using a jig is particularly desirable from the viewpoint of simplicity.

### <Fourth step 13>

The test piece which has been restrained after the loading of the external load in the fourth step 13 is placed for a predetermined time in a predetermined hydrogen entry environment. Then, in the fourth step 13, processing of assessing the occurrence state of cracking is performed with the test piece in that state.

At this time, the hydrogen entry environment and the placement time are preferably set to conditions under which a target hydrogen entry amount is achieved. The target hydrogen entry amount is the hydrogen entry amount equivalent to the amount of hydrogen that is estimated to enter in an environment in which the material to be assessed is actually used, for example.

The placement of the test piece in the hydrogen entry environment is performed by immersing the test piece in a bath containing an acid solution, such as hydrochloric acid or an aqueous NH₄SCN solution, for example. The concentration of the acid solution and the immersion time are set to achieve a condition under which hydrogen in the amount preset as the allowable upper limit enters the test piece.

For each test piece prepared in the first step 10, the second step 11 to the fourth step 13 above are carried out while the conditions of the forming strain to be applied and the load stress to be loaded are changed.

### <Fifth step 14>

In a fifth step 14, first, the limit load stress, which is a load stress at the limit where the delayed fracture does not occur in the sheared surface of the metal sheet, is assessed based on the results of the test above. Then, in the fifth step 14, the stress margin to the occurrence of the delayed fracture in the sheared end surface of the metal sheet is determined based on the limit load stress. Specifically, the limit load stress is set as the stress margin under the test conditions.

For example, the external load stress in which cracking occurs and a value of the limit load stress are determined based on the test conditions of each test piece and the assessment results of the occurrence or non-occurrence of cracking in the sheared end surface. The test conditions as used herein are the conditions of the forming strain and the external load stress set in the test. The external load stress in which cracking occurs refers to an external load stress, in which cracking occurs, to the same forming strain. The value of the limit load stress is a value of the limit load stress that is the boundary value with an external load stress in which no cracking occurs. The value of the limit load stress is the maximum value of the external load stress in which no cracking occurs, for example, or the like.

This is organized for a plurality of forming strains, and a plurality of pieces of data of forming strain and limit load stress is acquired. Then, the value (function) of the limit load stress with the forming strain as a variable is determined as data of the stress margin as represented by the graph in FIG. 2. More specifically, the stress margin is described as a function of the forming strain by tension and compression, for example.

Herein, the description above gives an example of the case where the test is performed while the forming strain is being changed, but the present invention is not limited thereto. The test may be carried out while the forming strain is limited to a constant forming strain (e.g., zero). This case also includes a case where the forming strain is zero, i.e., the second step 11 is not carried out. In this case, the stress margin to a specific forming strain is determined.

For example, it may be acceptable that the shearing conditions are changed, and the stress margin with the shearing conditions as variables may be determined together with the variable of the forming strain or without setting the forming strain as a variable. The shearing conditions are the clearance and the like.

The example illustrated in FIG. 2 is an example in which the residual stress of the sheared end surface decreases by the forming strain. A case in which the stress margin of the delayed fracture increases with an increase in the absolute value of the forming strain is illustrated as an example. However, it is also assumed that the stress margin of the delayed fracture conversely decreases by the forming strain, depending on the material or the state of the sheared end surface. This occurs due to the occurrence of cracking or damage in the sheared end surface by the forming strain to be applied, for example.

In this embodiment, a comparison with the stress margin determined as described above enables the following assessment. More specifically, a test piece having a sheared end surface, which was not used for the assessment of the stress margin, can also be assessed for a possibility of the delayed fracture without performing the test. The possibility of the delayed fracture is the possibility of the delayed fracture to the external load (load stress) to be load to the metal sheet.

For example, the assessment is performed as follows.

First, for the metal sheet to be assessed, the amount of strain by tension and compression and the load stress to the sheared end surface, where the occurrence of the delayed fracture is to be assessed, is calculated. The calculation is performed by a forming analysis by CAE or an elementary mechanical calculation method.

Next, the stress margin is associated corresponding to the amount of strain by tension and compression in the sheared end surface. Then, it is determined whether the load stress to the sheared end surface exceeds the stress margin. When the load stress to the sheared end surface exceeds the stress margin, it is determined that there is the possibility of the delayed fracture. At this time, the stress margin can also be set to be smaller than an actually measured value considering a safety factor.

The assessment of the delayed fracture in the sheared end surface, which is performed in comparison with the stress margin above, can also be more efficiently implemented by being performed by the program as described below.

The description above describes a case where no safety factor is set for the stress margin. However, it is possible to set the safety factor for the stress margin by setting the stress margin to be low.

The description above describes the stress margin as the value with the forming strain as a variable by actually applying the forming strain to the test piece in the second step 11 in the test. However, the present invention is not limited thereto.

For example, processing of applying bending processing to the sheared end surface of the metal sheet before the restraining step is provided. Thus, a step (referred to as a sixth step) of determining the forming residual stress generated in the sheared end surface and the forming strain generated in the sheared end surface is separately provided. The sixth step may be determined by an experiment that is separately carried out. The six step may be determined by a structural analysis, such as a CAE analysis. Then, the forming residual stress determined in the sixth step is added to the limit load stress (stress margin) determined by the test above in which the second step 11 is not performed. The value obtained by the addition may be set as the stress margin with the forming strain as a variable.

### (Details of disclosure)

The present disclosure is described in more detail.

The present inventors have found the following findings (1) to (3) during the assessment of the delayed fracture in the sheared end surface.
(1) A case is supposed in which a metal sheet is placed for a predetermined time in a hydrogen entry environment in a state where a load stress (external load stress) by a constant load is loaded to the sheared end surface and the metal sheet is restrained. In this case, for the load stress, there is a load stress at the limit where the delayed fracture does not occur in the sheared end surface (also referred to as the limit load stress). This is because the delayed fracture occurs when the total of the residual stress by shearing processing and the load stress from the outside in the sheared end surface reaches the threshold for the occurrence of the delayed fracture in the sheared end surface.
(2) The limit load stress in which the delayed fracture occurs changes with the amount of strain by tension and compression of the forming strain to be applied after shearing in the sheared end surface. This is because the residual stress of the sheared end surface changes by the forming strain.
(3) Accordingly, the limit load stress of each sheared end surface changes by the amount of the forming strain applied to the sheared end surface and the load stress (external load stress). When the metal sheet is placed for the predetermined placement time in the predetermined hydrogen entry environment, the load stress at the limit where the delayed fracture occurs in the sheared end surface can be summarized as follows. More specifically, the load stress at the limit can be summarized as an index of "stress margin" considering the amount of the forming strain and the load stress (the external load stress).

Herein, the present disclosure defines the allowance of the external load stress in which the delayed fracture does not occur possessed by the sheared end surface as the "stress margin".

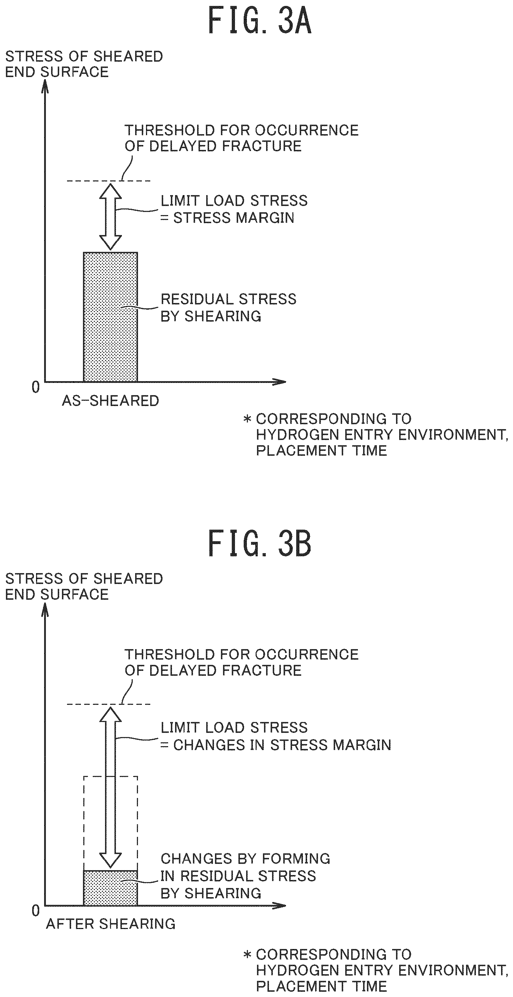

FIGS. 3A and 3B illustrate conceptual views for explaining (1), (2), (3) above. FIG. 3A illustrates the state of the load stress at the limit when no forming strain is applied, for a metal sheet having a sheared end surface formed by shearing an end part. On the other hand, FIG. 3B illustrates the state of the load stress at the limit when the forming strain is applied after the sheared end surface is formed. FIGS. 3A and 3B illustrate examples of a case where the residual stress decreases by applying the forming strain to the metal sheet before the metal sheet is press formed.

Herein, the delayed fracture occurs when the total of the residual stress by shearing and the load stress from the outside reaches the threshold for the occurrence of the delayed fracture. Accordingly, when the residual stress of the sheared end surface changes by the forming strain, the limit load stress in which the delayed fracture occurs also changes. The limit load stress is a difference between the residual stress of the sheared end surface and the threshold for the occurrence of the delayed fracture, and is the external load stress at the limit where the sheared end surface does not cause the delayed fracture.

In view of the above, the present disclosure defines the allowance of the external load stress in which the delayed fracture does not occur as the "stress margin" considering the forming strain to be applied to the sheared end surface. This embodiment prescribes the allowance of the external load stress by the index of the stress margin with the forming strain as a variable.

Herein, the residual stress in the sheared end surface by shearing is present only in a very small region of the extremely surface layer about 100 µm from the front surface of the sheared end surface. Therefore, changes in the residual stress are difficult to calculate by common CAE using a shell element or the like. Stress in microscopic regions can be measured by X-ray stress measurement or the like. However, there are such a problem that the measured value changes depending on the measurement range and such a problem that the measurement depth is limited to the top layer of a material. Accordingly, the large or small of the measured value sometimes does not necessarily correspond to a risk of the delayed fracture.

The correspondence can be achieved by the use of a technique of experimentally determining the "stress margin" above with the forming strain as a variable by a delayed fracture test in a stress loaded state. More specifically, an index for directly assessing the risk of the delayed fracture can be obtained without causing such problems with the calculation and the measurement.

It is supposed to assess the stress margin in a hydrogen entry environment condition to which automotive components are actually exposed. In this case, the stress margin itself can be regarded as the margin until the occurrence of the delayed fracture in the sheared end surfaces of the automotive components.

Further, the stress margin has the stress as the unit, and is expressed by the stress. Therefore, the stress margin can be achieved even when the external load stress to be applied to components in assembly, use, or the like, is added, in addition to the residual stress by the forming of the components. More specifically, it can be presumed that the delayed fracture does not occur unless the stress margin is exceeded.

Thus, the index of the stress margin is an excellent assessment index of the delayed fracture, the index which is simple and yet allows the assessment as the margin having a stress dimension.

On the contrary, a technique of changing the hydrogen entry environment while keeping the stress load value at a constant value can also be supposed. However, the technique has a problem with the addition of the external load stress by the deformation of components in assembly or use, for example, to the residual stress by the forming of the components described above. More specifically, the technique is less useful in that the assessment of the margin is impossible as compared with a case of using the stress as the standard.

The forming strain above is a strain in the extension direction in the sheared surface.

Further, with respect to practical assessment methods, the present inventors have found the following findings (4) to (7).

(4) As a method for introducing the forming strain of tension and compression to the sheared end surface into an assessment test piece, a uniaxial tensile deformation or a uniaxial compressive deformation is desirable. This is for the following reasons. In the uniaxial forming, springback after forming results in almost zero residual stress after forming in a portion other than the sheared end surface. Thus, it is because the influence is negligible. Accordingly, the application of the forming strain by the uniaxial tension or compression is advantageous in the following point. More specifically, the external load stress at the limit where the delayed fracture occurs in the sheared end surface after additional processing can be assessed as the "stress margin" as it is, which is the simplest.

(5) For a bending deformation to be introduced into actual press formed articles, the following can be said. More specifically, the "stress margin" in the sheared end surface after uniaxial tension or compression by the method (4) above can be obtained from the front surface to the back surface of a sheet. The obtained "stress margin" can be applied corresponding to the amount of strain and the sign of the strain. The "from the front surface to the back surface of the sheet" means "in the sheet thickness direction".

(6) The stress margin can also be assessed using a bending deformation as a method for applying the forming strain to the sheared end surface. In that case, there is a possibility that the bending deformation generates a residual stress by bending throughout the entire test piece including the sheared end surface. Therefore, in that case, the "stress margin" needs to be one obtained by adding the residual stress of the forming by bending to the external load stress at the limit where the delayed fracture occurs. The residual stress by the bending deformation can be assessed by a computer analysis by CAE or the like, elementary mechanical calculation, or the like.

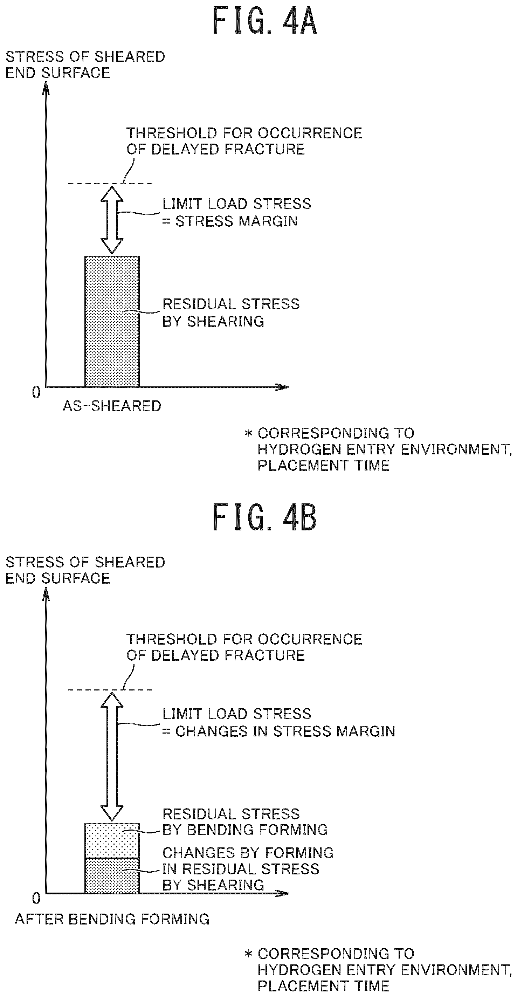

FIGS. 4A and 4B are conceptual views for explaining (6). FIGS. 4A and 4B illustrate cases where the residual stress by bending is positive. On the front and back sides of the sheet, the sign of the residual stress by bending is reversed. Accordingly, for a portion where the residual stress by bending is negative, a residual stress by shearing needs to be subtracted corresponding to the residual stress by bending forming. The subtraction means adding a negative value.

(7) A method for applying the external load stress to an assessment test piece includes a method using tension at a constant load or a method using bending. The method using bending is simple because the method can be implemented at a smaller load and using a smaller jig. The external load stress can be controlled by adjusting a load or a deformation amount to be applied to the test piece. The load to the sheared end surface of the test piece can be calculated by CAE or an elementary analysis.

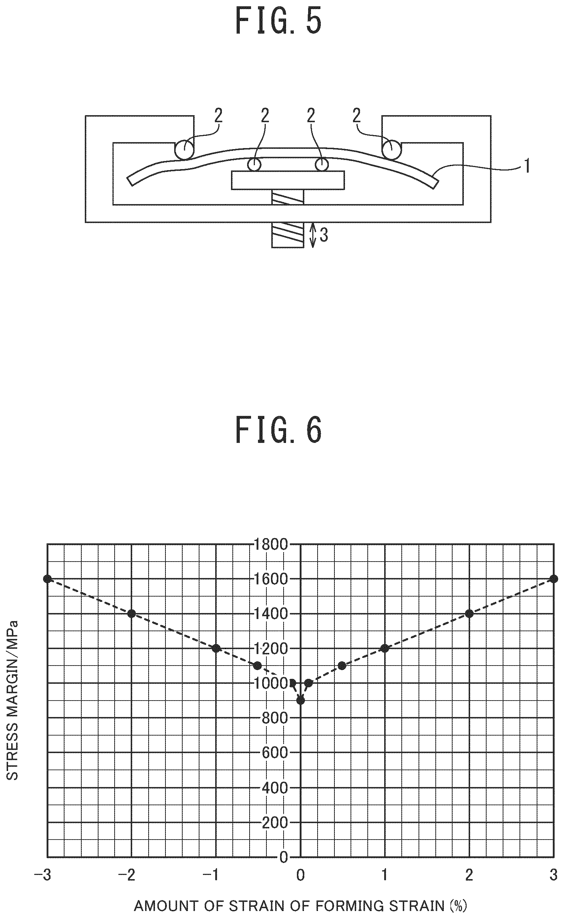

FIG. 5 is a view illustrating an example of the method for loading stress by bending in (7). FIG. 5 illustrates an example of a method for loading stress by four-point bending. In FIG. 5, the reference numeral 1 denotes a test piece. The reference numerals 2 denote supporting points where bending is loaded. The reference numeral 3 denotes a screw that adjusts the bending amount.

Herein, for the test piece to be assessed for the stress margin, a test piece obtained by laboratory-like shearing may be used. As the test piece, the sheared end surface of a press formed article after press forming may be partially cut out.

Further, the present inventors have devised the following technique as a method for assessing and predicting the occurrence of the delayed fracture in the sheared end surface using the "stress margin" thus obtained. An example of the method is described in the first to third items below.

### (First)

First, the stress margin is measured corresponding to the amount of strain by tension and compression applied to the test piece in a laboratory-like manner using the methods (4) to (7) above.

Herein, the hydrogen entry environment and the placement time in the environment are preferably set to such conditions that the amount of hydrogen entering the test piece is the target hydrogen entry amount. The target hydrogen entry amount is the amount of hydrogen preset as the allowable upper limit in actual automotive components.

As the amount of strain of the forming strain to be applied to the sheared end surface, the amount of strain is preferably set to 0.1% or more considering the amount having sufficient influence on the delayed fracture characteristics. As the amount having a greater degree of influence, the strain amount is 0.5% or more. When plastic strain is introduced, particularly the delayed fracture assessment according to the present invention is effective. Therefore, the amount of the plastic strain to the sheared end surface can be set as the assessment index in place of the forming strain. For the load stress to the test piece, any parameter related to stress, such as first principal stress or Mises stress, can be used in the present disclosure.

### (Second)

Second, a test piece having a sheared end surface to be assessed for the possibility of the delayed fracture is calculated for the amount of the forming strain by tension-compression and the load stress to the sheared end surface. The calculations are performed by a forming analysis by CAE or an elementary mechanical method, for example.

### (Third)

Third, a test piece having a sheared end surface to be assessed for the possibility of the delayed fracture is supposed. The amount of the forming strain and the residual stress after forming in the test piece are compared with the stress margin corresponding to the amount of strain by tension and compression. Then, a portion where the stress margin is exceeded is determined to have the possibility of the delayed fracture. However, the stress margin can also be set to be smaller than an actually measured value considering the safety factor.

### (Program)

An example of a program to be used for the above-described assessment method is described.

In a program of a first example, the relation between the stress margin and the forming strain determined by the method for assessing delayed fracture characteristics described above is stored in a storage unit. The program of the first example causes a computer to execute a reference to the stored relation between the stress margin and the forming strain above. Then, the program of the first example causes the computer to execute processing of determining the stress margin corresponding to the input amount of strain of the forming strain.

In a program of the other example, the relation between the stress margin, and the forming strain and the external load stress determined by the method for assessing delayed fracture characteristics described above is stored in a storage unit. The program of the other example causes a computer to execute a reference to the stored relation between the stress margin, and the forming strain and the external load stress above. Then, the program of the other example causes the computer to execute processing of assessing the possibility of the delayed fracture to the input amount of strain of the forming strain and the external load stress.

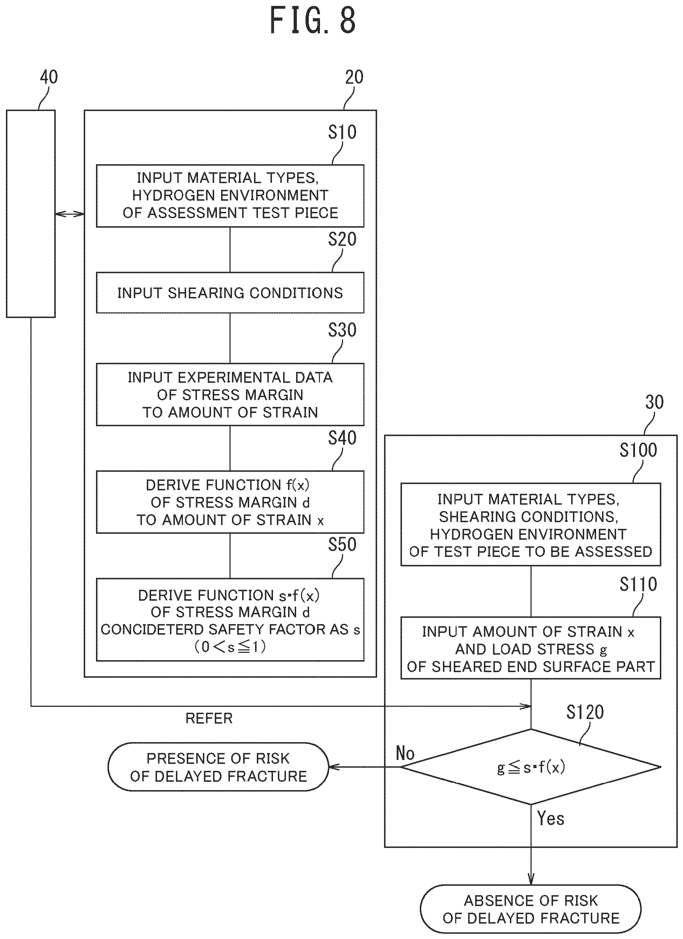

Next, an example of processing by the program of the assessment method using the stress margin described above is described with reference to FIG. 8. The assessment performed by the processing illustrated in FIG. 8 enables a more efficient assessment of the delayed fracture.

The example illustrated in FIG. 8 includes a stress margin calculation unit 20, an assessment main unit 30, and a storage unit 40. Programs for performing processing of the stress margin calculation unit 20 and the assessment main unit 30 are stored in the storage unit 40, such as a RAM or a ROM, of a computer and executed by the computer.

### <Storage unit 40>

The storage unit 40 includes a recording medium, such as a database.

The storage unit 40 stores data of a stress margin d determined to the forming strain with test conditions as variables for each of metal sheet material conditions, hydrogen environment conditions, and shearing conditions. The data are acquired by repeating the test from the first step 10 to the fifth step 14 while variously changing the metal sheet material conditions, the hydrogen environment conditions, the shearing conditions, and the amount of the forming strain.

### <Stress margin calculation unit 20>

The stress margin calculation unit 20 first prompts an input of the basic conditions of the assessment in Step S10, and then acquires the input by an input operation of an operator. The basic conditions of the assessment are material type (steel grade and thickness) conditions, hydrogen environment conditions (acidity and installation time), which are delayed fracture conditions, and the like.

Next, in Step S20, an input of the shearing conditions is promoted, and the input is acquired by an input operation of an operator.

Next, in Step S30, a data group matched with the conditions input in Step S10 and Step S20 are acquired from the storage unit 40. The data group is a data group of the stress margin to each amount of strain. The data group is also a set of data (amount of strain, stress margin).

Alternatively, an input of the data group of the stress margin to each amount of strain determined by the test is prompted, and the input information is acquired by an input operation of an operator. The acquired data are stored in the storage unit 40.

Next, in Step S40, the data group of the stress margin to the amount of strain acquired in Step S30 is referred to. Then, in Step S40, arithmetic processing of determining the stress margin d as a function f (x) with the amount of strain x as a variable is executed by a known processing system.

Next, in Step S50, the function of the stress margin d determined in Step S40 is changed to an equation considering a safety factor s (: 0 < s ≤ 1) as in the following equation. $d = s ⋅ f \left(x\right)$

Then, information of the function of the stress margin d determined above is stored in the storage unit 40 with the test conditions as a key.

### <Assessment main unit 30>

The assessment main unit 30 first prompts an input of material type conditions and hydrogen environment conditions, which are delayed fracture conditions, of an assessment target in Step S100. Then, the input is acquired by an input operation of an operator. The material types are a steel grade and a thickness, for example. The hydrogen environment conditions are acidity and installation time, for example.

Next, in Step S110, an input of the amount of strain x and a load stress g planned in processing is prompted, and the input is acquired by an input operation of an operator.

In Step S120, the information of the function "s·f (x)" of the stress margin d matched with the conditions input in Step S100 is acquired from the storage unit 40. Then, in Step S120, the stress margin d corresponding to the amount of strain x input in Step S110 and the load stress g input in Step S110 are compared with each other. Then, it is determined whether there is the risk of the delayed fracture based on the comparison.

Herein, in Step S120 in FIG. 8, it is determined whether there is the risk of the delayed fracture. The margin (= d - g) of the delayed fracture may be output together with the determination.

For the stress margin calculation unit 20, it may be acceptable that calculation processing is separately executed, and a function of the stress margin d with the input values in Steps S10 to S20 as conditions. Then, the determined function may be input in the storage unit 40 as data with the input values in Steps S10 to S20 as a key.

### EXAMPLES

Examples of this embodiment are described.

### (Example 1)

In this example, a description is given using a test material X containing a metal sheet being a 1470 MPa (tensile strength) class steel sheet having a thickness of 1.0 mm as a metal sheet to be assessed.

The present invention is not limited to the metal sheet of the test material. The present invention can be applied to various metal materials including high-strength steel sheets having a tensile strength of 980 MPa or more in which the delayed fracture occurs in a sheared end surface.

First, the test material X was sheared by shearing processing to prepare a test piece having a 100 mm long straight sheared end surface. The width of the test piece in shearing was set to 30 mm, and the test piece was formed into a 100 mm × 30 mm strip shape. A clearance in the shearing processing was set to 12% with respect to the sheet thickness. In the description in the embodiment above, the description is given taking the case where a single shearing condition is used as an example. However, when the shearing conditions, such as the clearance in the shearing processing, change, the assessment corresponding to the change is possible. More specifically, the stress margin under the shearing condition may be determined.

Next, a forming strain by tension or compression along the extension direction of the sheared end surface was applied to the sheared end surface of the test piece. In this example, the forming strain was applied by a uniaxial load testing machine with both ends of the test piece clamped. This example gives a description of the case where the forming strain is tension or compression. Even when the forming strain is a deformation by bending, it is confirmed that similar results are obtained. Further, a test piece that was kept as-sheared without applying the forming strain was also prepared.

Next, external restraint was applied to each test piece by four-point bending using a jig, and a stress was loaded to a center part of the sheared end surface of the test piece. A tensile stress was loaded with the burr side in shearing as the outside of the bending.

Herein, the magnitude of the load stress was determined as follows. A first principal stress-first principal strain relation in each of a width center part and a tip part of the test piece was determined by CAE. Then, the measurement was performed by measuring and associating the amount of strain when the test piece was actually bent.

In this example, the case of the four-point bending is described as a method for loading stress. By also the other bending loading methods and loading methods, such as uniaxial tension, results having similar tendency are obtained. In this example, a tensile stress was loaded with the burr side in shearing as the outside of the bending in stress loading. Similarly, the surface on the side opposite to the burr side can also be similarly assessed.

In this example, a load stress to be loaded to each test piece was changed in 100 MPa increments as shown in tables. Then, a plurality of test pieces was prepared for each forming strain condition.

The test pieces loaded with the load stress were immersed for 96 hours in a bath of a thiocyanate solution having a pH of 6. Then, the delayed fracture characteristics were assessed based on the occurrence or non-occurrence of cracking by the delayed fracture after 96 hours.

The conditions and the assessment results above are shown in Tables 1 to 11.

The tables are grouped for the amount of strain of the forming strain.

**[Table 1]**

| Amount of strain % | Load stress/MPa | Delayed fracture |
|---|---|---|
| | 500 | ○ |
| | 600 | ○ |
| | 700 | ○ |
| 0.0 | 800 | ○ |
| | 900 | ○ |
| | 1000 | × |
| | 1100 | × |

**[Table 2]**

| Amount of strain % | Load stress/MPa | Delayed fracture |
|---|---|---|
| | 600 | ○ |
| | 700 | ○ |
| | 800 | ○ |
| 0.1 | 900 | ○ |
| | 1000 | ○ |
| | 1100 | × |
| | 1200 | × |

**[Table 3]**

| Amount of strain % | Load stress/MPa | Delayed fracture |
|---|---|---|
| | 700 | ○ |
| | 800 | ○ |
| | 900 | ○ |
| 0.5 | 1000 | ○ |
| | 1100 | ○ |
| | 1200 | × |
| | 1300 | × |

**[Table 4]**

| Amount of strain % | Load stress/MPa | Delayed fracture |
|---|---|---|
| | 800 | ○ |
| | 900 | ○ |
| 1.0 | 1000 | ○ |
| | 1100 | ○ |
| | 1200 | ○ |
| | 1300 | × |
| | 1400 | × |

**[Table 5]**

| Amount of strain % | Load stress/MPa | Delayed fracture |
|---|---|---|
| | 1000 | ○ |
| | 1100 | ○ |
| | 1200 | ○ |
| 2.0 | 1300 | ○ |
| | 1400 | ○ |
| | 1500 | × |
| | 1600 | × |

**[Table 6]**

| Amount of strain % | Load stress/MPa | Delayed fracture |
|---|---|---|
| | 1200 | ○ |
| | 1300 | ○ |
| | 1400 | ○ |
| 3.0 | 1500 | ○ |
| | 1600 | ○ |
| | 1700 | × |
| | 1800 | × |

**[Table 7]**

| Amount of strain % | Load stress/MPa | Delayed fracture |
|---|---|---|
| | 600 | ○ |
| | 700 | ○ |
| | 800 | ○ |
| -0.1 | 900 | ○ |
| | 1000 | ○ |
| | 1100 | × |
| | 1200 | × |

**[Table 8]**

| Amount of strain % | Load stress/MPa | Delayed fracture |
|---|---|---|
| | 700 | ○ |
| | 800 | ○ |
| | 900 | ○ |
| -0.5 | 1000 | ○ |
| | 1100 | ○ |
| | 1200 | × |
| | 1300 | × |

**[Table 9]**

| Amount of strain % | Load stress/MPa | Delayed fracture |
|---|---|---|
| | 800 | ○ |
| | 900 | ○ |
| | 1000 | ○ |
| -1.0 | 1100 | ○ |
| | 1200 | ○ |
| | 1300 | × |
| | 1400 | × |

**[Table 10]**

| Amount of strain % | Load stress/MPa | Delayed fracture |
|---|---|---|
| | 1000 | ○ |
| | 1100 | ○ |
| | 1200 | ○ |
| -2.0 | 1300 | ○ |
| | 1400 | ○ |
| | 1500 | × |
| | 1600 | × |

**[Table 11]**

| Amount of strain % | Load stress/MPa | Delayed fracture |
|---|---|---|
| | 1200 | ○ |
| | 1300 | ○ |
| | 1400 | ○ |
| -3.0 | 1500 | ○ |
| | 1600 | ○ |
| | 1700 | × |
| | 1800 | × |

Each table shows the occurrence or non-occurrence of the delayed fracture for each load stress different depending on the amount of strain with the tension being positive and the compression being negative.

As is understood from Tables 1 to 11, the larger the absolute value of the amount of strain of the forming strain, the larger the limit load stress. In this example, the limit load stress with the forming strain as a variable is the stress margin.

In FIG. 6, the stress margin is determined from the load stress at the limit where the delayed fracture did not occur and the stress margin is illustrated as the function of the strain.

It is found that the stress margin corresponding to the forming strain after shearing can be described as described above. This example illustrates the case where the stress margin increases by the forming strain after shearing. Conversely, a similar assessment is possible also when the stress margin decreases by the forming strain after shearing.

### (Example 2)

Next, an example of the delayed fracture determination using the stress margin corresponding to the forming strain after shearing determined in Example 1 is described.

In Example 2, A, B, C, D, E, F, G, H test pieces were prepared which were test pieces prepared by applying steps similar to those of Example 1 to the test materials X and which were different in the amount of forming strain and load stress.

Each test piece was immersed for 96 hours in a bath of a thiocyanate solution having a pH of 6, and was assessed for the delayed fracture based on the occurrence or non-occurrence of cracking by the delayed fracture after 96 hours.

Table 12 shows the amount of the forming strain, the load stress, and the assessment results of the delayed fracture in the test pieces.

**[Table 12]**

| Test piece No. | Amount of forming strain % | Load stress/MPa | Delayed fracture |
|---|---|---|---|
| A | -0.9 | 600 | ○ |
| B | 0.8 | 1300 | × |
| C | 0.2 | 800 | ○ |
| D | -0.3 | 500 | ○ |
| E | 2.5 | 1300 | ○ |
| F | -1.6 | 700 | ○ |
| G | -1.1 | 1300 | × |
| H | 1.4 | 1000 | ○ |

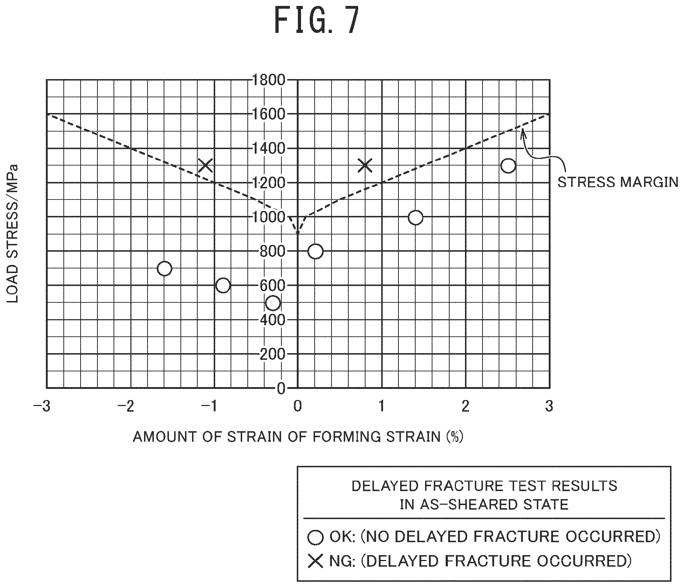

Next, the results of the test pieces A to G were plotted with the stress margins determined in Example 1 for comparison (see FIG. 6). FIG. 7 illustrates the comparison results.

As is understood from FIG. 7, it was found that the occurrence or non-occurrence of the delayed fracture in the sheared end surface in the actual components can be predicted based on whether the stress margin line is exceeded.

Accordingly, the use of the stress margin of the present disclosure has made it possible to determine a place having the risk of the delayed fracture in the sheared end surface.

The variable of the stress margin is used as the forming strain. However, the stress margin may be expressed with shearing conditions, such as the clearance of the shearing processing, as variables.

### Reference Signs List

10 first step (shearing step)
11 second step (step of applying forming strain)
12 third step (step of restraining in loaded state)
13 fourth step (step of placing for predetermined time in hydrogen entry environment)
14 fifth step (step of setting stress margin)
20 stress margin calculation unit
30 assessment main unit
40 storage unit
d stress margin

## Claims

1. A method for assessing delayed fracture characteristics for assessing delayed fracture characteristics of a sheared end surface of a metal sheet,
the method comprising:
a test including a step (12) of restraining the metal sheet in a state where a predetermined load stress is loaded to a sheared surface of the metal sheet and a step of placing the metal sheet for a predetermined time in a predetermined hydrogen entry environment in the restrained state; and
a step (14) of determining a limit load stress being a load stress at a limit where a delayed fracture in the sheared surface of the metal sheet does not occur based on results of the test and setting a stress margin (d) to occurrence of the delayed fracture in the sheared end surface of the metal sheet based on the determined limit load stress, wherein
the determined stress margin (d) is set as an index of the assessment of the delayed fracture characteristics of the sheared end surface of the metal sheet,
the test includes, before the restraining step (12), a step (11) of applying a forming strain along an extension direction of the sheared surface to the sheared surface of the metal sheet,
the stress margin (d) is set as a value with the forming strain as a variable, and
in the step (11) of applying the forming strain, the forming strain is applied by uniaxial tension or uniaxial compression.

2. The method for assessing delayed fracture characteristics of a sheared end surface according to claim **1,** wherein
the test is executed while selection conditions being one or two or more test conditions selected from test conditions other than the restraining step are changed, and the limit load stress under each selection condition is determined as the stress margin (d), and
the stress margin (d) is expressed as a value with the selection condition as a variable.

3. The method for assessing delayed fracture characteristics of a sheared end surface according to claim 1, wherein
the forming strain to be applied in the step of applying the forming strain is set to 0.1% or more.

4. The method for assessing delayed fracture characteristics of a sheared end surface according to claim 1 or 3, wherein
in the step (11) of applying the forming strain, the forming strain is applied by bending.

5. The method for assessing delayed fracture characteristics of a sheared end surface according to any one of claims 1 to 4, comprising:
a step of determining a forming residual stress generated in the sheared end surface by applying bending processing to the sheared end surface of the metal sheet before the restraining step, wherein
in the step (14) of setting the stress margin (d), a value obtained by adding the forming residual stress in the bending processing determined in the step of determining the forming residual stress to the determined limit load stress is set as the stress margin (d).

6. The method for assessing delayed fracture characteristics of a sheared end surface according to claim 5, wherein
in the step of determining the forming residual stress, a relation between the forming strain generated in the sheared end surface and the forming residual stress by the bending processing is determined, and
the stress margin (d) is set as a value with the forming strain as a variable.

7. A method for assessing delayed fracture characteristics of a sheared end surface for assessing a possibility of a delayed fracture in a sheared end surface of a metal sheet to be assessed being a metal sheet having same conditions as conditions of the metal sheet used in the test comprising:
assessing the possibility of the delayed fracture in an end surface of the metal sheet to be assessed using a forming strain and a load stress to be applied to the end surface of the metal sheet to be assessed based on the stress margin (d) determined by the method for assessing delayed fracture characteristics of a sheared end surface according to any one of claims 1, 3 or 4.

8. The method for assessing delayed fracture characteristics of a sheared end surface according to any one of claims 1 to 6, wherein
the metal sheet is a steel sheet having a tensile strength of 980 MPa or more.

9. A method for producing an automotive component from a metal sheet having a sheared end surface comprising:
predicting occurrence of the delayed fracture in the automotive component to be produced using the method for assessing delayed fracture characteristics of a sheared end surface according to any one of claims 1 to 8;
based on the prediction, selecting a shape or material of the metal sheet with less occurrence of the delayed fracture; and
producing the automotive component using the metal sheet with the selected shape or material.

## Patentansprüche

1. Verfahren zur Beurteilung von Merkmalen eines verzögerten Bruchs zur Beurteilung von Merkmalen eines verzögerten Bruchs einer gescherten Endfläche eines Metallblechs,
wobei das Verfahren Folgendes umfasst:
eine Prüfung, die einen Schritt (12) des Fixierens des Metallblechs in einem Zustand, in dem eine vorbestimmte Lastspannung auf eine gescherte Fläche des Metallblechs ausgeübt wird, und einen Schritt des Platzierens des Metallblechs für eine vorbestimmte Zeit in einer vorbestimmten Wasserstoffeintrittsumgebung in dem fixierten Zustand beinhaltet; und
einen Schritt (14) des Bestimmens einer Grenzlastspannung, die eine Lastspannung bei einer Grenze ist, bei der ein verzögerter Bruch in der gescherten Fläche des Metallblechs basierend auf Ergebnissen der Prüfung nicht auftritt, und des Festlegens einer Spannungsreserve (d) bei Auftreten des verzögerten Bruchs in der gescherten Endfläche des Metallblechs basierend auf der bestimmten Grenzlastspannung, wobei
die bestimmte Spannungsreserve (d) als Index für die Beurteilung der Merkmale eines verzögerten Bruchs der gescherten Endfläche des Metallblechs festgelegt ist,
die Prüfung vor dem Fixierschritt (12) einen Schritt (11) des Anwendens einer Umformdehnung entlang einer Ausdehnungsrichtung der gescherten Fläche auf die gescherte Fläche des Metallblechs beinhaltet,
die Spannungsreserve (d) als Wert festgelegt ist, bei dem die Umformdehnung eine Variable ist, und
die Umformdehnung in dem Schritt (11) des Anwendens der Umformdehnung durch einachsige Zugbeanspruchung oder einachsige Druckbeanspruchung angewendet wird.

2. Verfahren zur Beurteilung von Merkmalen eines verzögerten Bruchs einer gescherten Endfläche nach Anspruch 1, wobei die Prüfung ausgeführt wird, während Auswahlbedingungen, die eine oder zwei oder mehrere Prüfungsbedingungen sind, ausgewählt aus Prüfungsbedingungen, die nicht der Fixierschritt sind, geändert werden und die Grenzlastspannung unter jeder Auswahlbedingung als Spannungsreserve (d) bestimmt ist, und die Spannungsreserve (d) als Wert angegeben ist, bei dem die Auswahlbedingung eine Variable ist.

3. Verfahren zur Beurteilung von Merkmalen eines verzögerten Bruchs einer gescherten Endfläche nach Anspruch 1, wobei die in dem Schritt des Anwendens der Umformdehnung aufzuwendende Umformdehnung auf 0,1 % oder mehr festgelegt ist.

4. Verfahren zur Beurteilung von Merkmalen eines verzögerten Bruchs einer gescherten Endfläche nach Anspruch 1 oder 3, wobei die Umformdehnung in dem Schritt (11) des Anwendens der Umformdehnung durch Biegen angewendet ist.

5. Verfahren zur Beurteilung von Merkmalen eines verzögerten Bruchs einer gescherten Endfläche nach einem der Ansprüche 1 bis 4, umfassend:
einen Schritt des Bestimmens einer umformbedingten Eigenspannung, die in der gescherten Endfläche durch Anwenden eines Biegevorgangs auf die gescherte Endfläche des Metallblechs vor dem Fixierschritt erzeugt wird, wobei
in dem Schritt (14) des Festlegens der Spannungsreserve (d) als Spannungsreserve (d) ein Wert festgelegt wird, der durch Addition der in dem Schritt des Bestimmens der umformbedingten Eigenspannung bestimmten umformbedingten Eigenspannung aus dem Biegevorgang zu der bestimmten Grenzlastspannung erhalten wird.

6. Verfahren zur Beurteilung von Merkmalen eines verzögerten Bruchs einer gescherten Endfläche nach Anspruch 5, wobei in dem Schritt des Bestimmens der umformbedingten Eigenspannung eine Beziehung zwischen der in der gescherten Endfläche erzeugten Umformdehnung und der umformbedingten Eigenspannung durch den Biegevorgang bestimmt wird, und
die Spannungsreserve (d) als Wert festgelegt ist, bei dem die Umformdehnung eine Variable ist.

7. Verfahren zur Beurteilung von Merkmalen eines verzögerten Bruchs einer gescherten Endfläche zur Beurteilung einer Möglichkeit eines verzögerten Bruchs in einer gescherten Endfläche eines zu beurteilenden Metallblechs, das ein Metallblech ist, das dieselben Bedingungen wie die Bedingungen des bei der Prüfung verwendeten Metallblechs aufweist, umfassend:
Beurteilen der Möglichkeit des verzögerten Bruchs in einer Endfläche des zu beurteilenden Metallblechs unter Verwenden einer Umformdehnung und einer Lastspannung, die auf die Endfläche des zu beurteilenden Metallblechs anzuwenden sind, basierend auf der durch das Verfahren zur Beurteilung von Merkmalen eines verzögerten Bruchs einer gescherten Endfläche nach einem der Ansprüche 1, 3 oder 4 bestimmten Spannungsreserve (d) .

8. Verfahren zur Beurteilung von Merkmalen eines verzögerten Bruchs einer gescherten Endfläche nach einem der Ansprüche 1 bis 6, wobei
das Metallblech ein Stahlblech ist, das eine Zugfestigkeit von 980 MPa oder mehr aufweist.

9. Verfahren zur Herstellung einer Automobilkomponente aus einem Metallblech, das eine gescherte Endfläche aufweist, umfassend:
Prognostizieren eines Auftretens des verzögerten Bruchs in der herzustellenden Automobilkomponente unter Verwenden des Verfahrens zur Beurteilung von Merkmalen eines verzögerten Bruchs einer gescherten Endfläche nach einem der Ansprüche 1 bis 8;
basierend auf der Prognose Auswählen einer Form oder eines Materials des Metallblechs mit geringerem Auftreten des verzögerten Bruchs; und
Herstellen der Automobilkomponente unter Verwenden des Metallblechs mit der ausgewählten Form oder dem ausgewählten Material.

## Revendications

1. Procédé d'évaluation de caractéristiques de rupture différée pour l'évaluation de caractéristiques de rupture différée d'une surface d'extrémité cisaillée d'une tôle métallique,
le procédé comprenant :
un essai comportant une étape (12) de retenue de la tôle métallique dans un état où une contrainte de charge prédéterminée est chargée sur une surface cisaillée de la tôle métallique et une étape de placement de la tôle métallique pendant une durée prédéterminée dans un environnement d'entrée d'hydrogène prédéterminé dans l'état restreint ; et
une étape (14) de détermination d'une contrainte limite de charge étant une contrainte de charge au niveau d'une limite où une rupture différée de la surface cisaillée de la tôle métallique ne se produit pas, sur la base des résultats de l'essai, et de définition d'une marge de contrainte (d) pour l'apparition de la rupture différée de la surface d'extrémité cisaillée de la tôle métallique, sur la base de la contrainte limite de charge déterminée, dans lequel
la marge de contrainte déterminée (d) est définie comme un indice de l'évaluation de caractéristiques de rupture différée de la surface d'extrémité cisaillée de la tôle métallique,
l'essai comporte, avant l'étape de retenue (12), une étape (11) d'application d'une déformation de formage le long d'une direction d'extension de la surface cisaillée à la surface cisaillée de la tôle métallique,
la marge de contrainte (d) est définie comme une valeur avec la déformation de formage comme variable, et
à l'étape (11) d'application de la déformation de formage, la déformation de formage est appliquée par tension uniaxiale ou compression uniaxiale.

2. Procédé d'évaluation de caractéristiques de rupture différée d'une surface d'extrémité cisaillée selon la revendication 1, dans lequel
l'essai est exécuté tandis que les conditions de sélection, étant une ou deux conditions d'essai ou plus sélectionnées parmi les conditions d'essai autres que l'étape de retenue, sont modifiées, et la contrainte de charge limite sous chaque condition de sélection est déterminée comme la marge de contrainte (d), et
la marge de contrainte (d) est exprimée comme une valeur avec la condition de sélection comme variable.

3. Procédé d'évaluation de caractéristiques de rupture différée d'une surface d'extrémité cisaillée selon la revendication 1, dans lequel
la déformation de formage à appliquer à l'étape d'application de la déformation de formage est fixée à 0,1 % ou plus.

4. Procédé d'évaluation de caractéristiques de rupture différée d'une surface d'extrémité cisaillée selon la revendication 1 ou 3, dans lequel
à l'étape (11) d'application de la déformation de formage, la déformation de formage est appliquée par pliage.

5. Procédé d'évaluation de caractéristiques de rupture différée d'une surface d'extrémité cisaillée selon l'une quelconque des revendications 1 à 4, comprenant :
une étape de détermination d'une contrainte résiduelle de formage générée dans la surface d'extrémité cisaillée par l'application d'un traitement de pliage à la surface d'extrémité cisaillée de la tôle métallique avant l'étape de retenue, dans lequel
à l'étape (14) de définition de la marge de contrainte (d), une valeur obtenue par l'ajout de la contrainte résiduelle de formage dans le processus de pliage déterminée à l'étape de détermination de la contrainte résiduelle de formage à la contrainte de charge limite déterminée est définie comme la marge de contrainte (d).

6. Procédé d'évaluation de caractéristiques de rupture différée d'une surface d'extrémité cisaillée selon la revendication 5, dans lequel
à l'étape de détermination de contrainte résiduelle de formage, une relation entre la déformation de formage générée à la surface d'extrémité cisaillée et la contrainte résiduelle de formage par le traitement de pliage est déterminée, et
la marge de contrainte (d) est définie comme une valeur avec la déformation de formage comme variable.

7. Procédé d'évaluation de caractéristiques de rupture différée d'une surface d'extrémité cisaillée pour l'évaluation d'une possibilité d'une rupture différée dans une surface d'extrémité cisaillée d'une tôle métallique à évaluer ayant les mêmes conditions que les conditions de la tôle métallique utilisée lors de l'essai, comprenant :
l'évaluation de la possibilité de la rupture différée dans une surface d'extrémité de la tôle métallique à évaluer à l'aide d'une déformation de formage et d'une contrainte de charge à appliquer à la surface d'extrémité de la tôle métallique à évaluer sur la base de la marge de contrainte (d) déterminée par le procédé d'évaluation de caractéristiques de rupture différée d'une surface d'extrémité cisaillée selon l'une quelconque des revendications 1, 3 ou 4.

8. Procédé d'évaluation de caractéristiques de rupture différée d'une surface d'extrémité cisaillée selon l'une quelconque des revendications 1 à 6, dans lequel
la tôle métallique est une tôle d'acier ayant une résistance à la traction de 980 MPa ou plus.

9. Procédé de fabrication d'un composant automobile à partir d'une tôle métallique ayant une surface d'extrémité cisaillée, comprenant :
la prédiction de l'apparition de la rupture différée dans le composant automobile à produire à l'aide du procédé d'évaluation de caractéristiques de rupture différée d'une surface d'extrémité cisaillée selon l'une quelconque des revendications 1 à 8 ;
sur la base de la prédiction, la sélection d'une forme ou d'un matériau pour la tôle métallique avec moins de risques de la rupture différée ; et
la production du composant automobile à l'aide de la tôle métallique avec la forme ou le matériau sélectionné.
